# EUROPEAN PATENT SPECIFICATION

(11) **EP 1 553 985 B1**
(45) Date of publication and mention of the grant of the patent: **31.08.2011**
(21) Application number: 03808140.2
(22) Date of filing: 06.10.2003
(51) Int. Cl.: A61K 51/04

(54) **BIPHENYLS AS IMAGING AGENTS IN ALZHEIMER'S DISEASE**
BIPHENYLE ALS BILDERZEUGUNGSMITTEL BEI DER ALZHEIMERSCHEN KRANKHEIT
BIPHENYLES COMME AGENTS D'IMAGERIE POUR LA MALADIE D'ALZHEIMER

(30) Priority: 04.10.2002 US 415824 P
(43) Date of publication of application: 20.07.2005
(73) Proprietor: The Trustees of The University of Pennsylvania, Philadelphia, Pennsylvania 19104-6283 (US)
(72) Inventor: Kung, Hank.F., Wynnewood, PA 19096 (US); Kung, Mei Ping., Wynnewood, PA 19096 (US); Zhang, Zhi-Ping, Lansdale, PA 19446 (US)
(74) Representative: Luderschmidt, Schüler & Partner
(86) International application number: PCT/US2003/031466
(87) International publication number: WO 2004/032975

(56) References cited:
- WO-A-93/24115
- WO-A-98/47969
- WO-A-99/41224
- AU-B- 545 825
- US-A- 2 157 071
- US-A- 3 821 293
- US-A- 5 739 166
- LEE C-W ET AL: "Dimethylamino-fluorenes: ligands for detecting beta-amyloid plaques in the brain" NUCLEAR MEDICINE AND BIOLOGY, ELSEVIER SCIENCE PUBLISHERS, NEW YORK, NY, US, vol. 30, no. 6, August 2003 (2003-08), pages 573-580, XP004444175 ISSN: 0969-8051
- DATABASE CHEMABS [Online] CHEMICAL ABSTRACTS SERVICE, COLUMBUS, OHIO, US; REISSERT, ARNOLD: "Attempts to prepare substituted indigos" XP002270065 retrieved from STN Database accession no. 8:10644 & BER. , 47, 672-81, 1914,
- DATABASE CHEMABS [Online] CHEMICAL ABSTRACTS SERVICE, COLUMBUS, OHIO, US; VORLANDER, D.: "The doctrine of intramolecular oppositions and the directing of substituents in benzene. II" XP002270066 retrieved from STN Database accession no. 20:4855 & BER. , 58B, 1893-914, 1925,
- DATABASE CHEMABS [Online] CHEMICAL ABSTRACTS SERVICE, COLUMBUS, OHIO, US; THEILACKER, WALTER ET AL: "Dye theory. Triarylmethane dyes of the biphenyl series. II" XP002270004 retrieved from STN Database accession no. 51:17167 & CHEMISCHE BERICHTE , 89, 970-83 CODEN: CHBEAM; ISSN: 0009-2940, 1956,
- DATABASE CHEMABS [Online] CHEMICAL ABSTRACTS SERVICE, COLUMBUS, OHIO, US; BARKER, C. C. ET AL: "Steric effects in di- and triarylmethanes. VII. Biphenyl analogs of crystal violet and malachite green" XP002270067 retrieved from STN Database accession no. 56:18064 & JOURNAL OF THE CHEMICAL SOCIETY, ABSTRACTS 3445-8 CODEN: JCSAAZ; ISSN: 0590-9791, 1961,
- KLUNK W E ET AL: "DEVELOPMENT OF SMALL MOLECULE PROBES FOR THE BETA-AMYLOID PROTEIN OF ALZHEIMER'S DISEASE" NEUROBIOLOGY OF AGING, TARRYTOWN, NY, US, vol. 15, no. 6, 1994, pages 691-698, XP000602786 ISSN: 0197-4580

## Description

### BACKGROUND OF THE INVENTION

### Field of the Invention

This invention relates to novel bioactive compounds, pharmaceutical and diagnostic composition for imaging amyloid deposits and the use of the novel compounds.

### Background Art

Alzheimer's disease (AD) is a progressive neurodegenerative disorder characterized by cognitive decline, irreversible memory loss, disorientation, and language impairment. Postmortem examination of AD brain sections reveals abundant senile plaques (SPs) composed of amyloid-β (Aβ) peptides and numerous neurofibrillary tangles (NFTs) formed by filaments of highly phosphorylated tau proteins (for recent reviews and additional citations see Ginsberg, S. D., et al., "Molecular Pathology of Alzheimer's Disease and Related Disorders," in Cerebral Cortex: Neurodegenerative and Age-related Changes in Structure and Function of Cerebral Cortex, Kluwer Academic/Plenum, NY (1999), pp. 603-654; Vogelsberg-Ragaglia, V., et al., "Cell Biology of Tau and Cytoskeletal Pathology in Alzheimer's Disease," Alzheimer's Disease, Lippincot, Williams & Wilkins, Philadelphia, PA (1999), pp. 359-372). Familial AD (FAD) is caused by multiple mutations in the A precursor protein (APP), presenilin 1 (PS1) and presenilin 2 (PS2) genes (Ginsberg, S. D., et al., "Molecular Pathology of Alzheimer's Disease and Related Disorders," in Cerebral Cortex: Neurodegenerative and Age-related Changes in Structure and Function of Cerebral Cortex, Kluwer Academic/Plenum, NY (1999), pp. 603-654; Vogelsberg-Ragaglia, V., et al., "Cell Biology of Tau and Cytoskeletal Pathology in Alzheimer's Disease," Alzheimer's Disease, Lippincot, Williams & Wilkins, Philadelphia, PA (1999), pp. 359-372).

While the exact mechanisms underlying AD are not fully understood, all pathogenic FAD mutations studied thus far increase production of the more amyloidogenic 42-43 amino-acid long form of the Aβ peptide. Thus, at least in FAD, dysregulation of Aβ production appears to be sufficient to induce a cascade of events leading to neurodegeneration. Indeed, the amyloid cascade hypothesis suggests that formation of extracellular fibrillar Aβ aggregates in the brain may be a pivotal event in AD pathogenesis (Selkoe, D. J., "Biology of B-amyloid Precursor Protein and the Mechanism of Alzheimer's Disease," Alzheimer's Disease, Lippincot Williams & Wilkins, Philadelphia, PA (1999), pp. 293-310; Selkoe, D. J., J. Am. Med. Assoc. 283:1615-1617 (2000); Naslund, J., et al., J. Am. Med. Assoc. 283:1571-1577 (2000); Golde, T. E., et al., Biochimica et Biophysica Acta 1502:172-187 (2000)).

Various approaches in trying to inhibit the production and reduce the accumulation of fibrillar Aβ in the brain are currently being evaluated as potential therapies for AD (Skovronsky, D. M. and Lee, V. M., Trends Pharmacol. Sci. 21:161-163 (2000); Vassar, R., et al., Science 286:735-741 (1999); Wolfe, M. S., et al., J. Med. Chem. 41:6-9 (1998); Moore, C. L., et al., J. Med. Chem. 43:3434-3442 (2000); Findeis, M. A., Biochimica et Biophysica Acta 1502:76-84 (2000); Kuner, P., Bohrmann, et al., J. Biol. Chem. 275:1673-1678 (2000)). It is therefore of great interest to develop ligands that specifically bind fibrillar Aβ aggregates. Since extracellular SPs are accessible targets, these new ligands could be used as *in vivo* diagnostic tools and as probes to visualize the progressive deposition of Aβ in studies of AD amyloidogenesis in living patients.

To this end, several interesting approaches for developing fibrillar Aβ aggregate-specific ligands have been reported (Ashburn, T. T., et al., Chem. Biol. 3:351-358 (1996); Han, G., et al., J. Am. Chem. Soc. 118:4506-4507 (1996); Klunk, W. E., et al., Biol. Psychiatry 35:627 (1994); Klunk, W. E., et al., Neurobiol. Aging 16:541-548 (1995); Klunk, W. E., et al., Society for Neuroscience Abstract 23:1638 (1997); Mathis, C. A., et al., Proc. XIIth Intl. Symp. Radiopharm. Chem., Uppsala, Sweden:94-95 (1997); Lorenzo, A. and Yankner, B. A., Proc. Natl. Acad. Sci. U.S.A. 91:12243-12247 (1994); Zhen, W., et al., J. Med. Chem. 42:2805-2815 (1999)). One approach is based on highly conjugated chrysamine-G (CG) and Congo red (CR), and the latter has been used for fluorescent staining of SPs and NFTs in postmortem AD brain sections (Ashburn, T. T., et al., Chem; Biol. 3:351-358 (1996); Klunk, W. E., et al., J Histochem. Cytochem. 37:1273-1281 (1989)). The inhibition constants (Kᵢ) for binding to fibrillar Aβ aggregates of CR, CG, and 3'-bromo- and 3'-iodo derivatives of CG are reported to be 2,800, 370, 300 and 250 nM, respectively (Mathis, C. A., et al., Proc. XIIth Intl. Symp. Radiopharm. Chem., Uppsala, Sweden:94-95 (1997)). These compounds have been shown to bind selectively to Aβ (1-40) peptide aggregates *in vitro* as well as to fibrillar Aβ deposits in AD brain sections (Mathis, C. A., et al., Proc. XIIth Intl. Symp. Radiopharm. Chem., Uppsala, Sweden:94-95 (1997)).

Amyloidosis is a condition characterized by the accumulation of various insoluble, fibrillar proteins in the tissues of a patient. An amyloid deposit is formed by the aggregation of amyloid proteins, followed by the further combination of aggregates and/or amyloid proteins. Formation and accumulation of aggregates of β-amyloid (Aβ) peptides in the brain are critical factors in the development and progression of AD. The fibrillar aggregates of amyloid peptides, Aβ₁₋₄₀ and Aβ₁₋₄₂, are major metabolic peptides derived from amyloid precursor protein found in senile plaques and cerebrovascular amyloid deposits in AD patients (Xia, W., et al., J. Proc. Natl. Acad. Sci. U.S.A. 97:9299-9304 (2000)). Prevention and reversal of Aβ plaque formation are being targeted as a treatment for this disease (Selkoe, D., J. JAMA 283:1615-1617 (2000); Wolfe, M.S., et al., J. Med. Chem. 41:6-9 (1998); Skovronsky, D.M., and Lee, V.M., Trends Pharmacol. Sci. 21:161-163 (2000)).

In addition to the role of amyloid deposits in Alzheimer's disease, the presence of amyloid deposits has been shown in diseases such as Mediterranean fever, Muckle-Wells syndrome, idiopathetic myeloma, amyloid polyneuropathy, amyloid cardiomyopathy, systemic senile amyloidosis, amyloid polyneuropathy, hereditary cerebral hemorrhage with amyloidosis, Down's syndrome, Scrapie, Creutzfeldt-Jacob disease, Kuru, Gerstamnn-Straussler-Scheinker syndrome, medullary carcinoma of the thyroid, Isolated atrial amyloid, β₂-microglobulin amyloid in dialysis patients, inclusion body myositis, β₂-amyloid deposits in muscle wasting disease, and Islets of Langerhans diabetes Type II insulinoma.

Thus, a simple, noninvasive method for detecting and quantitating amyloid deposits in a patient has been eagerly sought. Presently, detection of amyloid deposits involves histological analysis of biopsy or autopsy materials. Both methods have drawbacks. For example, an autopsy can only be used for a postmortem diagnosis.

Imaging agents may be based on two types of isotopes. ^{99m}Tc (T_{1/2}, 6 h; 140 KeV) and ¹²³I (T_{1/2}, 13 h; 159 KeV) are routinely used for single photon emission computed tomography (SPECT), while ¹¹C (T_{1/5}, 20 min; 511 KeV) and ¹⁸F (T_{1/2}, 110 min; 511 KeV) are commonly used for positron emission tomography (PET).

The direct imaging of amyloid deposits *in vivo* is difficult, as the deposits have many of the same physical properties (e.g., density and water content) as normal tissues. Attempts to image amyloid deposits using magnetic resonance imaging (MRI) and computer-assisted tomography (CAT) have been disappointing and have detected amyloid deposits only under certain favorable conditions. In addition, efforts to label amyloid deposits with antibodies, serum amyloid P protein, or other probe molecules have provided some selectivity on the periphery of tissues, but have provided for poor imaging of tissue interiors.

Potential ligands for detecting Aβ aggregates in the living brain must cross the intact blood-brain barrier. Thus brain uptake can be improved by using ligands with relatively smaller molecular size (compared to Congo Red) and increased lipophilicity. Highly conjugated thioflavins (S and T) are commonly used as dyes for staining the Aβ aggregates in the AD brain (Elhaddaoui, A., et al., Biospectroscopy 1: 351-356 (1995)). These compounds are based on benzothiazole, which is relatively small in molecular size.

It would be useful to have a noninvasive technique for imaging and quantitating amyloid deposits in a patient. In addition, it would be useful to have compounds that inhibit the aggregation of amyloid proteins to form amyloid deposits and a method for determining a compound's ability to inhibit amyloid protein aggregation.

### BRIEF SUMMARY OF THE INVENTION

The present invention provides novel compounds of Formula I.
The present invention also provides a pharmaceutical composition comprising a compound of Formula I.

The present invention further provides diagnostic compositions comprising a radiolabeled compound of Formula I and a pharmaceutically acceptable carrier or diluent.

A further aspect of this invention is directed to the use of compounds of Formula I in the manufacture of a diagnostic composition for imaging amyloid deposits.

### BRIEF DESCRIPTION OF THE FIGURES

Figure 1 depicts the structures of potential Aβ plaque imaging agents.

Figure 2 depicts the binding data for some of the biphenyl and pyrazole compounds of the present invention.

### DETAILED DESCRIPTION OF THE INVENTION

A first aspect of the present invention is directed to compounds of Formula I: 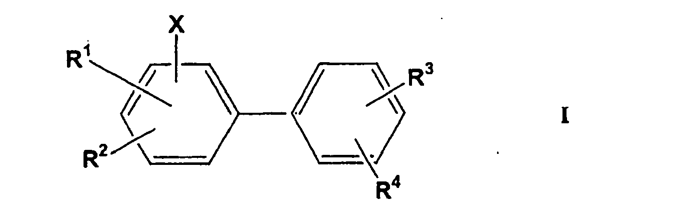 or a pharmaceutically acceptable salt thereof, wherein

R¹ is dimethylamino, R² is hydrogen, R³ is ¹⁸fluoro (C₁₋₅)alkyl , R⁴ is hydroxy and X is hydrogen. In these embodiments, it is more preferred that R¹ and R⁴ are in the para position relative to the bridge, and R³ is in the ortho position relative to R⁴. Most preferably, R³ is ¹⁸fluoromethyl or ¹⁸fluoroethyl.

Some preferred compounds falling under general Formula I have the following structures i-iii: 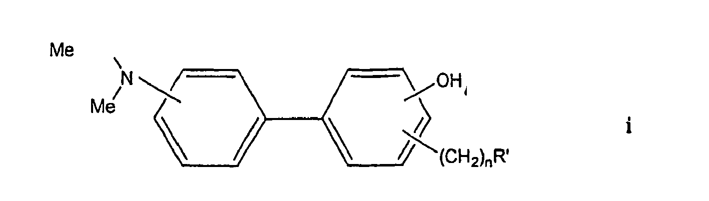 Where, n= 1 -5 and R' = ¹⁸F. 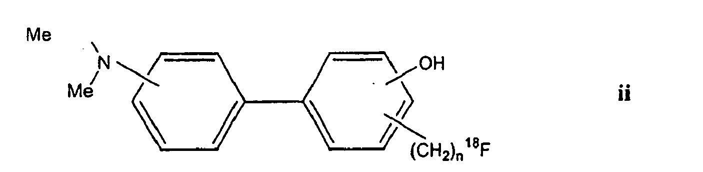 Where, n=1-5.

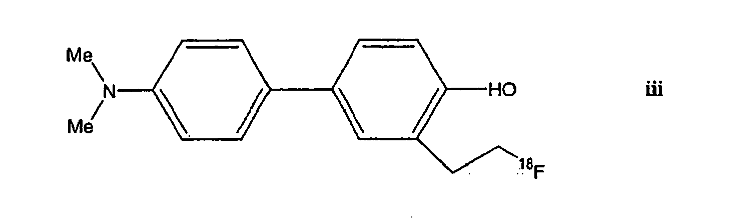

With respect to the relative positions of any substituent on an aromatic ring, it is envisioned that R¹, R², R³, R⁴ and X may occur at ortho, meta, or para positions relative to the linkage bond between the aromatic rings. It is also envisioned that in preferred embodiments wherein each aromatic ring has one substituent, the ortho, meta or para position of each substituent is independent of the substituent on the opposite ring. In compounds containing one substituent on each ring it is preferred that each substituent is independently either in a meta or para position relative to said linkage bond. Most preferably, both substituents are in the para position.

It is also to be understood that the present invention is considered to include stereoisomers as well as optical isomers, e.g. mixtures of enantiomers as well as individual enantiomers and diastereomers, which arise as a consequence of structural asymmetry in selected compounds of the present series.

The compounds of Formula I may also be solvated, especially hydrated. Hydration may occur during manufacturing of the compounds or compositions comprising the compounds, or the hydration may occur over time due to the hygroscopic nature of the compounds. In addition, the compounds of the present invention can exist in unsolvated as well as solvated forms with pharmaceutically acceptable solvents such as water, ethanol, and the like. In general, the solvated forms are considered equivalent to the unsolvated forms for the purposes of the present invention.

When any variable occurs more than one time in any constituent or in Formula I its definition on each occurrence is independent of its definition at every other occurrence. Also combinations of substituents and/or variables are permissible only if such combinations result in stable compounds.

The term "alkyl" as employed herein by itself or as part of another group refers to both straight and branched chain radicals of up to 8 carbons, preferably 6 carbons, more preferably 4 carbons, such as methyl, ethyl, propyl, isopropyl, butyl, t-butyl, and isobutyl.

The term "alkoxy" is used herein to mean a straight or branched chain alkyl radical, as defined above, unless the chain length is limited thereto, bonded to an oxygen atom, including, but not limited to, methoxy, ethoxy, n-propoxy, isopropoxy, and the like. Preferably the alkoxy chain is 1 to 6 carbon atoms in length, more preferably 1-4 carbon atoms in length.

The term "monoalkylamine" as employed herein by itself or as part of another group refers to an amino group which is substituted with one alkyl group as defined above. Thus, the term "methylamino" refers to a neutral group or ring substituent, wherein the N is connected to a compound of one of the general formulas disclosed herein via the ring or a chain of the compound, wherein the N is further bound to a methyl and a hydrogen. Further, the N may be charged and may form salts.

The term "dialkylamine" as employed herein by itself or as part of another group refers to an amino group which is substituted with two alkyl groups as defined above. Thus, the term "dimethylamino" refers to a neutral group or ring substituent, wherein the N is connected to a compound of one of the general formulas disclosed herein via the ring or a chain of the compound, wherein the N is further bound to two methyl groups. Further, the N may be charged and may form salts.

The term "hydroxy(C₁₋₅)alkyl" as employed herein refers to an alkyl chain connected to a compound of one of the general formulas disclosed herein via the ring or a chain of the compound, wherein the distal portion of the alkyl chain of the group contains a hydroxy moiety. The alkyl chain can contain any number of carbons, but preferably the number of carbons in the alkyl chain is from 1 to 5.

The term "halo" employed herein by itself or as part of another group refers to chlorine, bromine, fluorine or iodine.

The term "haloalkyl" as employed herein refers to any of the above alkyl groups substituted by one or more chlorine, bromine, fluorine or iodine with fluorine and chlorine being preferred, such as chloromethyl, iodomethyl, trifluoromethyl, 2,2,2-trifluoroethyl, and 2-chloroethyl.

The term "alkylthio" as employed herein by itself or as part of another group refers to a thioether of the structure: R-S, wherein R is a C₁₋₄ alkyl as defined above.

The term "alkylsulfonyl" as employed herein by itself or as part of another group refers to a sulfone of the structure: R-SO₂, wherein R is a C₁₋₄ alkyl as defined above.

The term "aryl" as employed herein by itself or as part of another group refers to monocyclic or bicyclic aromatic groups containing from 6 to 12 carbons in the ring portion, preferably 6-10 carbons in the ring portion, such as phenyl, naphthyl or tetrahydronaphthyl.

The term "heterocycle" or "heterocyclic ring", as used herein except where noted, represents a stable 5- to 7- membered mono-heterocyclic ring system which may be saturated or unsaturated, and which consists of carbon atoms and from one to three heteroatoms selected from the group consisting, of N, O, and S, and wherein the nitrogen and sulfur heteroatom may optionally be oxidized. Especially useful are rings contain one nitrogen combined with one oxygen or sulfur, or two nitrogen heteroatoms. Examples of such heterocyclic groups include piperidinyl, pyrrolyl, pyrrolidinyl, imidazolyl, imidazinyl, imidazolidinyl, pyridyl, pyrazinyl, pyrimidinyl, oxazolyl, oxazolidinyl, isoxazolyl, isoxazolidinyl, thiazolyl, thiazolidinyl, isothiazolyl, homopiperidinyl, homopiperazinyl, pyridazinyl, pyrazolyl, and pyrazolidinyl, most preferably thiamorpholinyl, piperazinyl, and morpholinyl.

The term "heteroatom" is used herein to mean an oxygen atom ("O"), a sulfur atom ("S") or a nitrogen atom ("N"). It will be recognized that when the heteroatom is nitrogen, it may form an NR^{a}R^{b} moiety, wherein R^{a} and R^{b} are, independently from one another, hydrogen or C₁₋₄ alkyl, C₂₋₄ aminoalkyl, C₁₋₄ halo alkyl, halo benzyl, or R¹ and R² are taken together to form a 5- to 7-member heterocyclic ring optionally having O, S or NR^{c} in said ring, where R^{c} is hydrogen or C₁₋₄ alkyl.

The term "heteroaryl" as employed herein refers to groups having 5 to 14 ring atoms; 6, 10 or 14 π electrons shared in a cyclic array; and containing carbon atoms and 1, 2 or 3 oxygen, nitrogen or sulfur heteroatoms (where examples of heteroaryl groups are: thienyl, benzo[b]thienyl, naphtho[2,3-b]thienyl, thianthrenyl, furyl, pyranyl, isobenzofuranyl, benzoxazolyl, chromenyl, xanthenyl, phenoxathiinyl, 2H-pyrrolyl, pyrrolyl, imidazolyl, pyrazolyl, pyridyl, pyrazinyl, pyrimidinyl, pyridazinyl, indolizinyl, isoindolyl, 3H-indolyl, indolyl, indazolyl, purinyl, 4H-quinolizinyl, isoquinolyl, quinolyl, phthalazinyl, naphthyridinyl, quinazolinyl, cinnolinyl, pteridinyl, 4aH-carbazolyl, carbazolyl, β-carbolinyl, phenanthridinyl, acridinyl, perimidinyl, phenanthrolinyl, phenazinyl, isothiazolyl, phenothiazinyl, isoxazolyl, furazanyl and phenoxazinyl groups).

The term "aralkyl" or "arylalkyl" as employed herein by itself or as part of another group refers to C₁₋₆alkyl groups as discussed above having an aryl substituent, such as benzyl, phenylethyl or 2-naphthylmethyl.

Another aspect of this invention is related to methods of preparing compounds of Formula I.

The present invention is further directed to methods of preparing compounds of the above Formula I. All reagents used in the synthesis were commercial products and were used without further purification unless otherwise indicated. Anhydrous Na₂SO₄ was used as a drying agent. Flash column chromatography was performed on 230-400 mesh silica gel.

The compounds of this invention can be prepared by reactions described in Schemes 1-6. Synthesis of N,N-dimethylamino derivatives of fluorene was successfully achieved by a reductive methylation reaction shown in Scheme 1. Starting with 2- or 3-aminofluorenes, 1a-1f, the amino group was converted to the N,N-dimethylamino group (2a-2f) in excellent yield (>90%) using paraformaldehyde in the presence of sodium cyanoborohydride as a reducing agent (4). A same reaction was applied in the methylation of 9-fluorenones (Scheme 2) by which the amino-9-fluorenones were converted to N,N-dimethylamino-9-hydroxyfluorenes (3a-3d) in good yield (>80%). Under the reductive methylation condition, the keto group of fluorenone was reduced to 9-hydroxy group. To preserve the keto group of the 9-fluorenone, an alternative method was employed for the methylation reaction. Using methyliodide/K₂CO₃ in refluxing acetonitrile, the amino group of 9-fluorenone was methylated to the N,N-dimethylamino-9-fluorenones (4a-4d) (Scheme 3). The yields for this methylation reaction were less predictable (ranging from 18-70%)_{.}
Scheme 5 and 6 depict a synthetic route for preparing biphenyl compounds of Formula I.

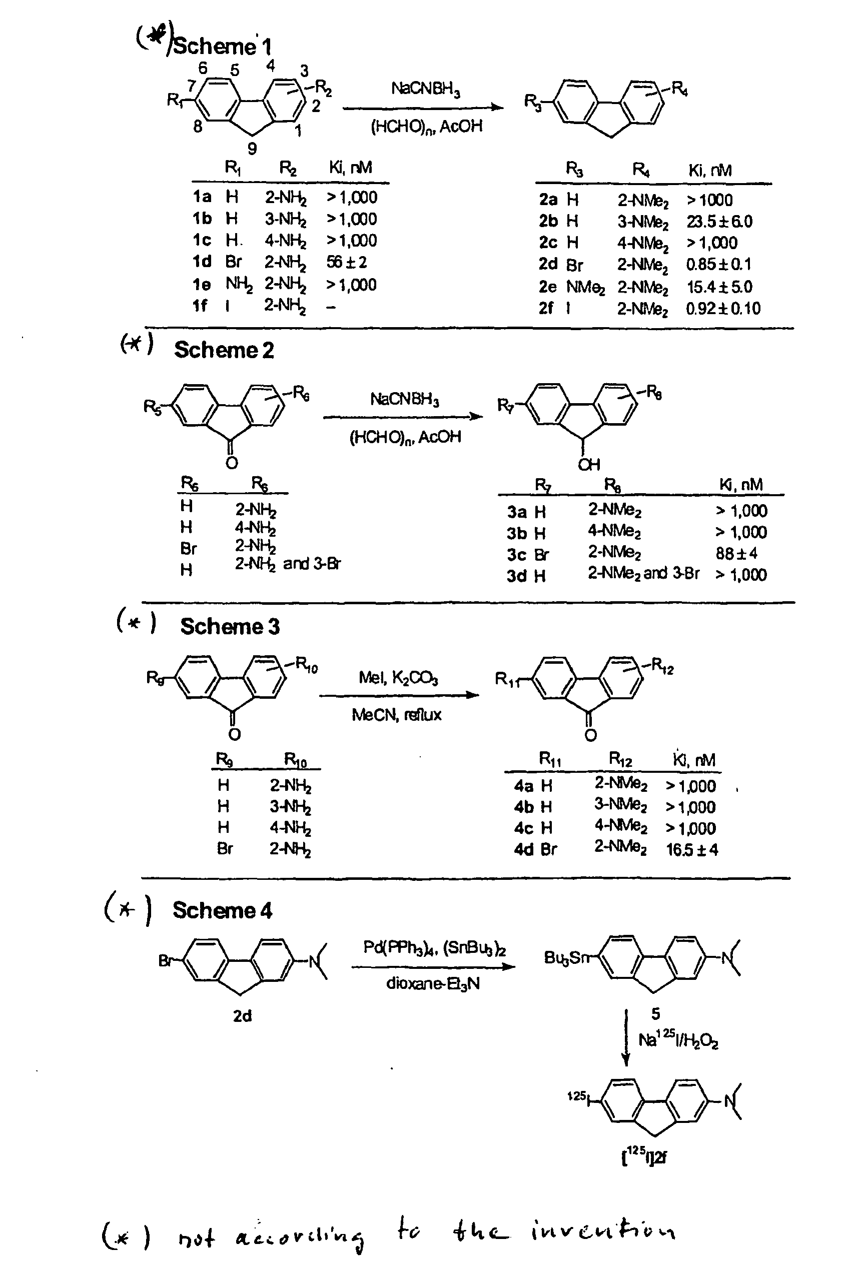

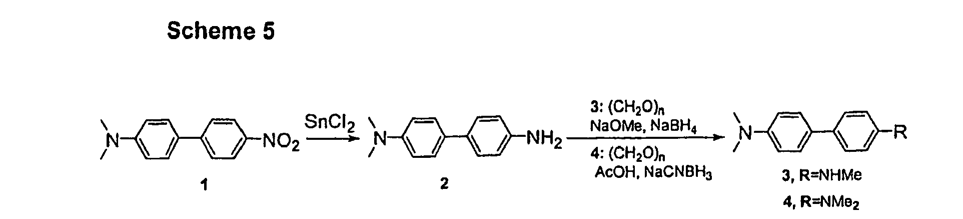

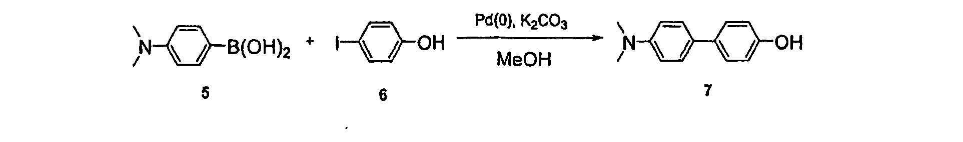

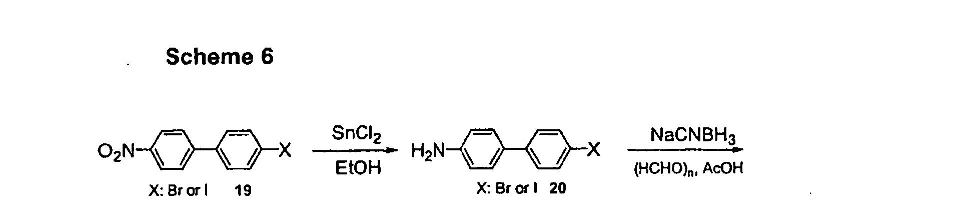

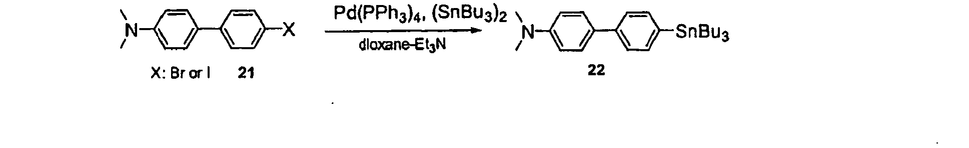

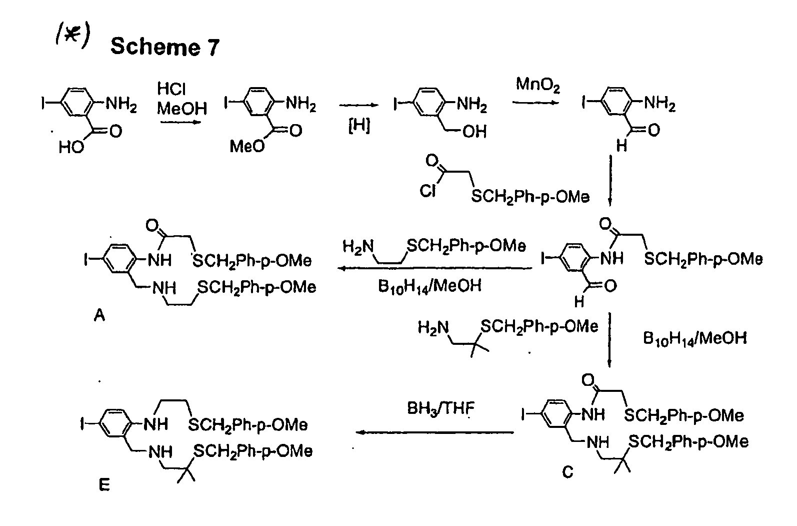

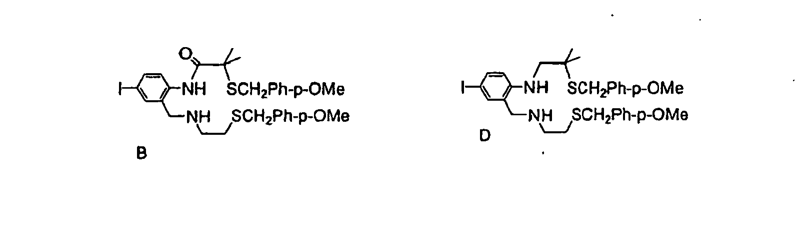

(*) not according to the invention 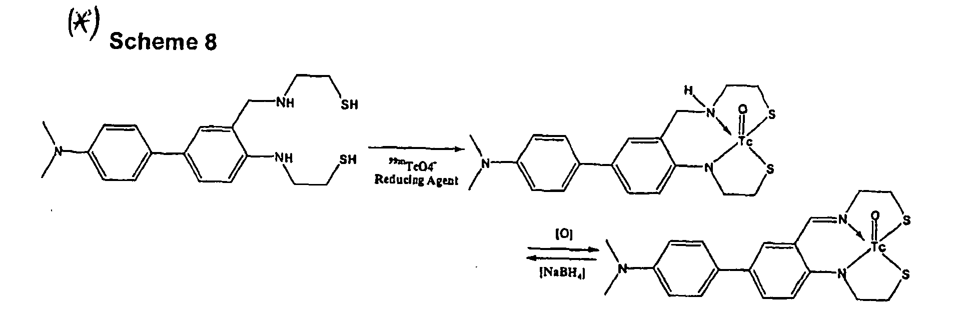 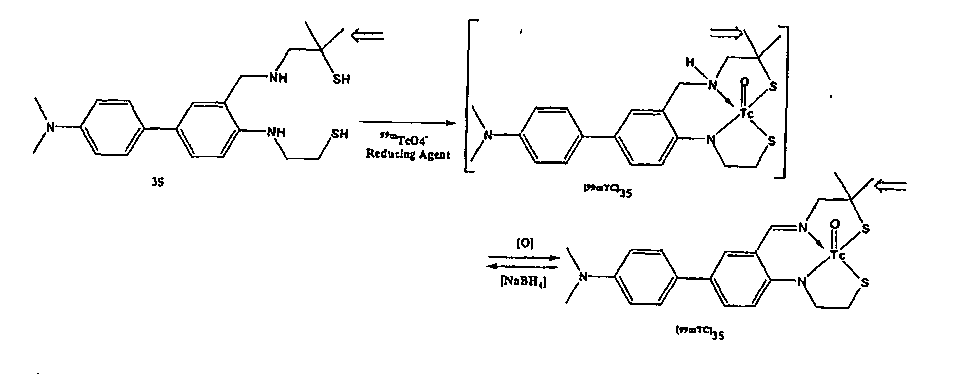 (*) not according to the invention 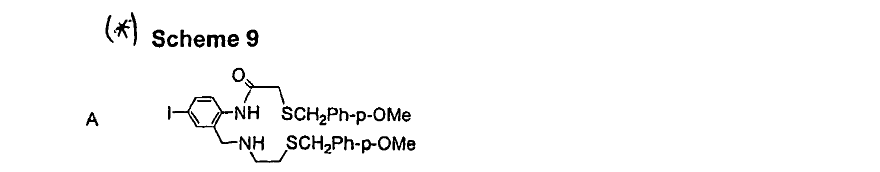 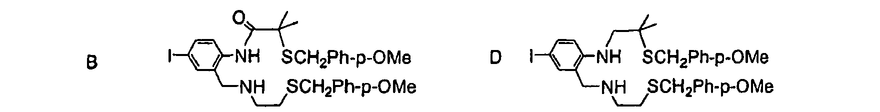 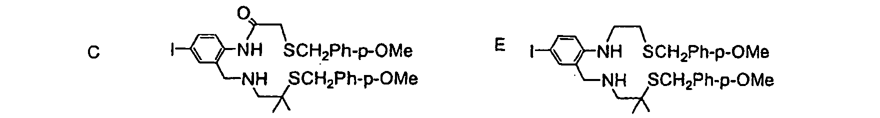 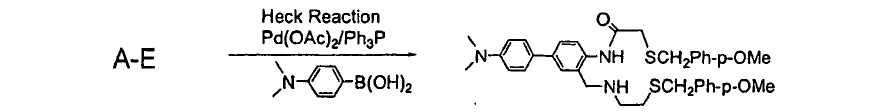 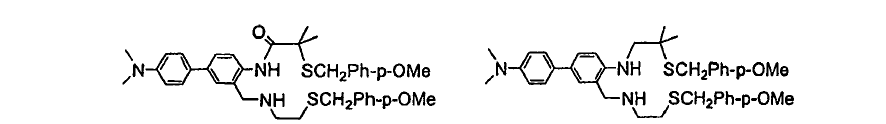 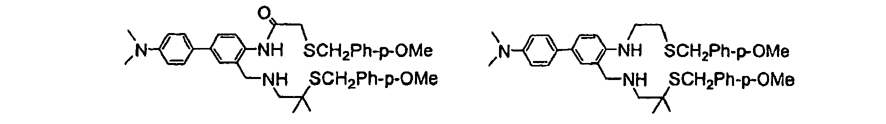 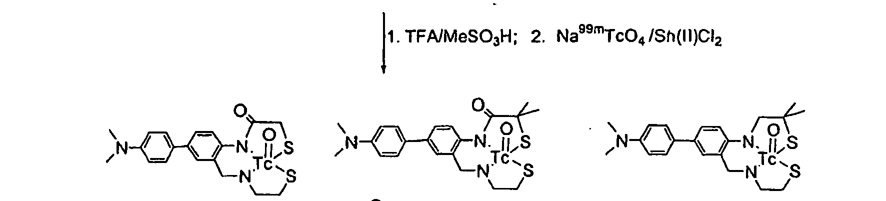 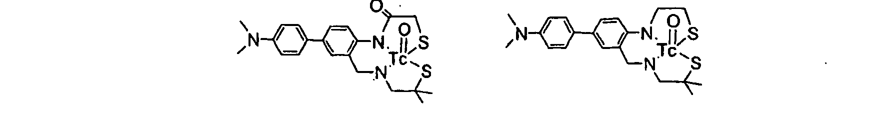 (*) not according to the invention 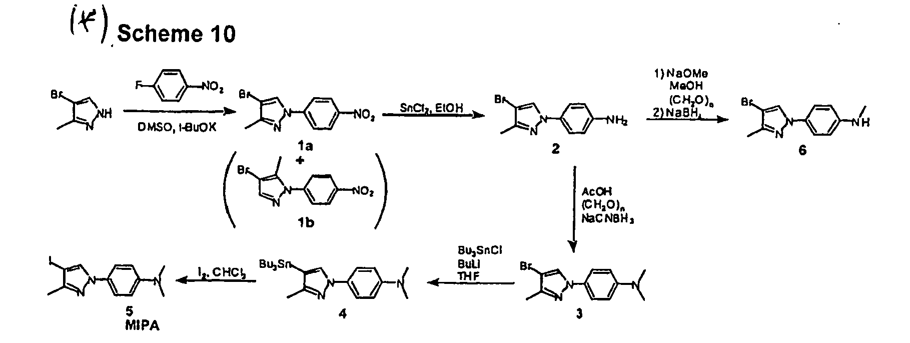 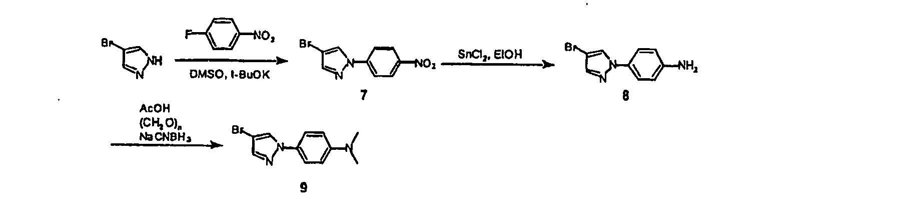 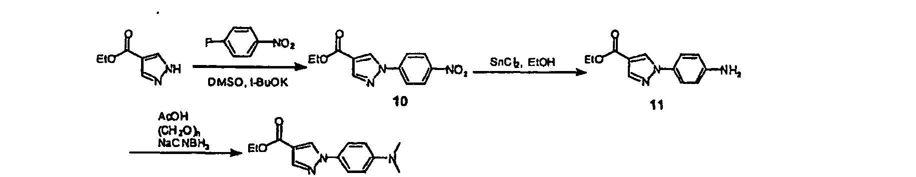 (*) not according to the invention [0091] When the compounds of this invention are to be used as imaging agents, they must be labeled with suitable radioactive halogen isotopes.

The radiohalogenated compounds of this invention lend themselves easily to formation from materials which could be provided to users in kits. Kits for forming the imaging agents can contain, for example, a vial containing a physiologically suitable solution of an intennediate of Formula I in a concentration and at a pH suitable for optimal complexing conditions. The user would add to the vial an appropriate quantity of the radioisotope, e.g., Na¹²³I, and an oxidant, such as hydrogen peroxide. The resulting labeled ligand may then be administered intravenously to a patient, and receptors in the brain imaged by means of measuring the gamma ray or photo emissions therefrom.

Since the radiopharmaceutical composition according to the present invention can be prepared easily and simply, the preparation can be carried out readily by the user. Therefore, the present invention also relates to a kit, comprising:
(1) A non-radiolabeled compound of the invention, the compound optionally being in a dry condition; and also optionally having an inert, pharmaceutically acceptable carrier and/or auxiliary substances added thereto; and
(2) a reducing agent and optionally a chelator; wherein ingredients (1) and (2) may optionally be combined; and further wherein instructions for use with a prescription for carrying out the above-described method by reacting ingredients (1) and (2) with technetium-99m in the form of a pertechnetate solution may be optionally included.

Examples of suitable reducing agents and chelators for the above kit have been listed above. The pertechnetate solution can be obtained by the user from a molybdenum-technetium generator. Such generators are available in a number of institutions that perform radiodiagnostic procedures. As noted above the ingredients (1) and (2) may be combined, provided they are compatible. Such a monocomponent kit, in which the combined ingredients are preferably lyophilized, is excellently suitable to be reacted by the user with the pertechnetate solution in a simple manner.

When desired, the radioactive diagnostic agent may contain any additive such as pH controlling agents (e.g., acids, bases, buffers), stabilizers (e.g., ascorbic acid) or isotonizing agents (e.g., sodium chloride).

The term "pharmaceutically acceptable salt" as used herein refers to those carboxylate salts or acid addition salts of the compounds of the present invention which are, within the scope of sound medical judgement, suitable for use in contact with the tissues of patients without undue toxicity, irritation, allergic response, and the like, commensurate with a reasonable benefit/risk ratio, and effective for their intended use, as well as the zwitterionic forms, where possible, of the compounds of the invention. The term "salts" refers to the relatively nontoxic, inorganic and organic acid addition salts of compounds of the present invention. Also included are those salts derived from non-toxic organic acids such as aliphatic mono and dicarboxylic acids, for example acetic acid, phenyl-substituted alkanoic acids, hydroxy alkanoic and alkanedioic acids, aromatic acids, and aliphatic and aromatic sulfonic acids. These salts can be prepared *in situ* during the final isolation and purification of the compounds or by separately reacting the purified compound in its free base form with a suitable organic or inorganic acid and isolating the salt thus formed. Further representative salts include the hydrobromide, hydrochloride, sulfate, bisulfate, nitrate, acetate, oxalate, valerate, oleate, palmitate, stearate, laurate, borate, benzoate, lactate, phosphate, tosylate, citrate, maleate, fumarate, succinate, tartrate, naphthylate mesylate, glucoheptonate, lactiobionate and laurylsulphonate salts, propionate, pivalate, cyclamate, isethionate, and the like. These may include cations based on the alkali and alkaline earth metals, such as sodium, lithium, potassium, calcium, magnesium, and the like, as well as, nontoxic ammonium, quaternary ammonium and amine cations including, but not limited to ammonium, tetramethylammonium, tetraethylammonium, methylamine, dimethylamine, trimethylamine, triethylamine, ethylamine, and the like. (See, for example, Berge S. M., et al., Pharmaceutical Salts, J. Pharm. Sci. 66:1-19 (1977)

In the first step of the present method of imaging, a labeled compound of Formula I is introduced into a tissue or a patient in a detectable quantity. The compound is typically part of a pharmaceutical composition and is administered to the tissue or the patient by methods well known to those skilled in the art.

For example, the compound can be administered either orally, rectally, parenterally (intravenous, by intramuscularly or subcutaneously), intracistemally, intravaginally, intraperitoneally, intravesically, locally (powders, ointments or drops), or as a buccal or nasal spray.

In the framework of the invention, the labeled compound is introduced into a patient in a detectable quantity and after sufficient time has passed for the compound to become associated with amyloid deposits, the labeled compound is detected noninvasively inside the patient. Alternatively, a labeled compound of Formula I is introduced into a patient, sufficient time is allowed for the compound to become associated with amyloid deposits, and then a sample of tissue from the patient is removed and the labeled compound in the tissue is detected apart from the patient. Also alternatively, a tissue sample is removed from a patient and a labeled compound of Formula I is introduced into the tissue sample. After a sufficient amount of time for the compound to become bound to amyloid deposits, the compound is detected.

The administration of the labeled compound to a patient can be by a general or local administration route. For example, the labeled compound may be administered to the patient such that it is delivered throughout the body. Alternatively, the labeled compound can be administered to a specific organ or tissue of interest. For example, it is desirable to locate and quantitate amyloid deposits in the brain in order to diagnose or track the progress of Alzheimer's disease in a patient.

The term "tissue" means a part of a patient's body. Examples of tissues include the brain, heart, liver, blood vessels, and arteries. A detectable quantity is a quantity of labeled compound necessary to be detected by the detection method chosen. The amount of a labeled compound to be introduced into a patient in order to provide for detection can readily be determined by those skilled in the art. For example, increasing amounts of the labeled compound can be given to a patient until the compound is detected by the detection method of choice. A label is introduced into the compounds to provide for detection of the compounds.

The term "patient" means humans and other animals. Those skilled in the art are also familiar with determining the amount of time sufficient for a compound to become associated with amyloid deposits. The amount of time necessary can easily be determined by introducing a detectable amount of a labeled compound of Formula I, II, III, IV, V or VI into a patient and then detecting the labeled compound at various times after administration.

The term "associated" means a chemical interaction between the labeled compound and the amyloid deposit. Examples of associations include covalent bonds, ionic bonds, hydrophilic-hydrophilic interactions, hydrophobic-hydrophobic interactions, and complexes.

Those skilled in the art are familiar with the various ways to detect labeled compounds. For example, magnetic resonance imaging (MRI), positron emission tomography (PET), or single photon emission computed tomography (SPECT) can be used to detect radiolabeled compounds. The label that is introduced into the compound will depend on the detection method desired. For example, if PET is selected as a detection method, the compound must possess a positron-emitting atom, such as ¹¹C or ¹⁸F.

The radioactive diagnostic agent should have sufficient radioactivity and radioactivity concentration which can assure reliable diagnosis. For instance, in case of the radioactive metal being technetium-99m, it may be included usually in an amount of 0.1 to 50 mCi in about 0.5 to 5.0 ml at the time of administration. The amount of a compound of Formula I, may be such as sufficient to form a stable chelate compound with the radioactive metal.

The thus formed chelate compound as a radioactive diagnostic agent is sufficiently stable, and therefore it may be immediately administered as such or stored until its use. When desired, the radioactive diagnostic agent may contain any additive such as pH controlling agents (e.g., acids, bases, buffers), stabilizers (e.g., ascorbic acid) or isotonizing agents (e.g., sodium chloride).

The imaging of amyloid deposits can also be carried out quantitatively so that the amount of amyloid deposits can be determined.

Preferred compounds for imaging include the radioisotope ¹⁸F .

The present invention is also directed at the use of a compound of general Formula I in the manufacture of a diagnostic composition for imaging amyloid deposits. One of the key prerequisites for an *in vivo* imaging agent of the brain is the ability to cross the intact blood-brain barrier after a bolus iv injection.

## Claims

1. A compound of general Formula I: 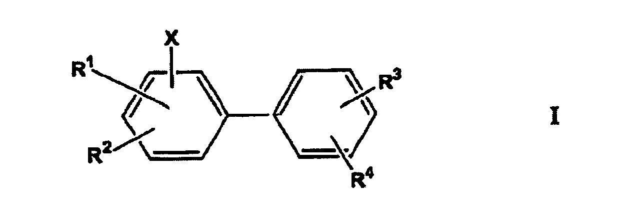 or a pharmaceutically acceptable salt thereof, wherein
R¹ is dimethylamino,
R² is hydrogen,
R³ is ¹⁸fluoro(C₁₋₅)alkyl,
R⁴ is hydroxyl, and
X is hydrogen.

2. The compound of claim 1, wherein
R³ is ¹⁸ fluoromethyl or ¹⁸fluoroethyl.

3. The compound of claim 2, wherein
R³ is ¹⁸fluoroethyl.

4. A pharmaceutical composition comprising a compound of any one of claims 1-3.

5. A diagnostic composition for use in imaging amyloid deposits, comprising a radiolabeled compound of any one of claims 1-3; and a pharmaceutically acceptable excipient or diluent.

6. Use of the compounds of claim 1 in the manufacture of a diagnostic composition for imaging amyloid deposits.

## Patentansprüche

1. Verbindung der allgemeinen Formel I: 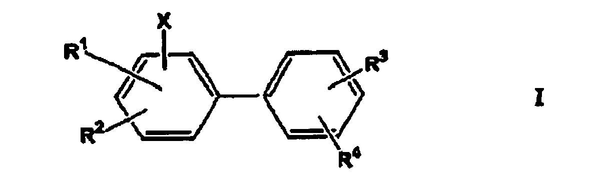 oder ein pharmazeutisch zulässiges Salz davon, wobei
R¹ Dimethylamino ist,
R² Wasserstoff ist,
R^{3 18}Fluor(C₁₋₅)alkyl,
R⁴ Hydroxyl ist, und
X Wasserstoff ist.

2. Verbindung nach Anspruch 1, wobei:
R^{3 18}Fluormethyl oder ¹⁸Fluorethyl ist.

3. Verbindung nach Anspruch 2, wobei
R^{3 18}Fluorethyl ist.

4. Pharmazeutische Zusammensetzung, umfassend eine Verbindung nach einem der Ansprüche 1-3.

5. Diagnostische Zusammensetzung zur Verwendung bei der bildgebenden Diagnostik von Amyloid-Ablagerungen, umfassend eine radioaktiv markierte Verbindung nach einem der Ansprüche 1-3 und einen pharmazeutisch wirksamen Hilfsstoff oder ein pharmazeutisch wirksames Verdünnungsmittel.

6. Verwendung der Verbindungen nach Anspruch 1 bei der Herstellung einer diagnostischen Zusammensetzung zur bildgebenden Diagnostik von Amyloid-Ablagerungen.

## Revendications

1. Composé de formule générale I : 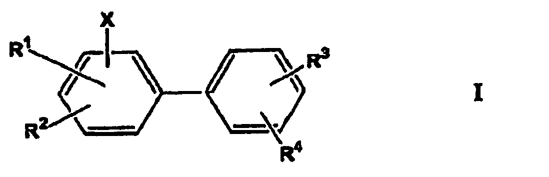 ou sel pharmaceutiquement acceptable de celui-ci, où
R1 est diméthylamino,
R2 est l'hydrogène,
R3 est 18fluoro(C1-5)alkyle,
R4 est hydroxyle, et
X est l'hydrogène.

2. Composé selon la revendication 1 où
R3 est 18fluorométhyle ou 18 fluoroéthyle.

3. Composé selon la revendication 2 où
R3 est 18fluoroéthyle.

4. Composition pharmaceutique comprenant un composé selon l'une quelconque des revendications 1-3.

5. Composition diagnostique destinée à être utilisée dans l'imagerie des dépôts amyloïdes comprenant un composé radiomarqué selon l'une quelconque des revendications 1-3 ; et un excipient ou diluant pharmaceutiquement acceptable.

6. Utilisation des composés selon la revendication 1 dans la fabrication d'une composition diagnostique pour l'imagerie des dépôts amyloïdes.
